# EUROPEAN PATENT SPECIFICATION

(11) **EP 4 056 117 B1**
(45) Date of publication and mention of the grant of the patent: **11.06.2025**
(21) Application number: 21162348.3
(22) Date of filing: 12.03.2021
(51) Int. Cl.: A61B 5/25, A61B 5/00

(54) **MEDICAL ELECTRODE**
MEDIZINISCHE ELEKTRODE
ÉLECTRODE MÉDICALE

(43) Date of publication of application: 14.09.2022
(73) Proprietor: Ambu A/S, 2750 Ballerup (DK)
(72) Inventor: DAMMEYER, Marie, Riis, 2750 Ballerup (DK)
(74) Representative: Guardian IP Consulting I/S

(56) References cited:
- EP-A2- 0 210 020
- US-A1- 2005 261 565
- US-A1- 2015 011 857

## Description

The present disclosure relates to a medical electrode including a plastic foam disc having a top side and a bottom side, an aperture extending at a central part of the plastic foam disc from the top side to the bottom side and thereby forming a contact medium chamber, a plastic foil having a top side and a bottom side and being arranged on the top side of the plastic foam disc, thereby covering the aperture and at least a part of the surrounding plastic foam disc, the plastic foil being welded to the plastic foam disc along a weld line surrounding the aperture, an electric connector being mounted on the plastic foil eccentrically in relation to the central part of the plastic foam disc, the electric connector being adapted to be electrically connected to monitoring equipment, a sensor strip disposed in the contact medium chamber and electrically connected to the electric connector, and a layer of pressure-sensitive adhesive applied to the bottom side of the plastic foam disc.

Such electrodes are used for establishing electrical contact between the skin of humans or animals and electrical measuring equipment for measuring or detecting bioelectrical signals caused by physiological processes, such as the heart function. Plotting of curves indicative of this function is called electrocardiography, abbreviated as ECG, and electrodes of this type are therefore often called ECG electrodes.

JP 3574943 B2 discloses a biomedical electrode using a conductive adhesive gel layer for attaching the electrode to the skin. The conductive adhesive gel layer is covered by an air-permeable protective member and the electrode is provided with a centrally arranged electrical connector for connection to monitoring equipment. The electrode is furthermore provided with a ring-formed reinforcing member arranged on the top of the air-permeable protective member, at the rim of the conductive adhesive gel layer in order to suppress deformation of the electrode and in order to suppress the occurrence of displacement between the protective member and the adhesive gel layer caused by the water absorption and the adhesion of the gel to the clothes or the like.

WO 95/28877 (Medicotest A/S) discloses a medical electrode comprising a sensor which is attached to a person's skin. Spaced from the sensor, the electrode is provided with a relief which is also adhered to the person's skin. The electrode is shaped in such a manner between the sensor and the attachment point as to permit and compensate for mutual movements between the sensor and the attachment point, without such movements loading the sensor.

US 4,640,289 discloses a biomedical electrode including a conductive metal layer arranged under a retainer sheet. In the preferred embodiment, the retainer sheet and metal layer are preattached to each other, and the retainer sheet is comprised of a gravure printed, silver/silver chloride treated, polyester film. The polyester film is a dielectric material that can insulate the remainder of the electrode from stray electrical signals that could otherwise be picked up from the surroundings including static charges from nearby clothing, bedding or the like.

US 4,524,775 (Medicotest A/S) discloses a medical electrode for contacting the skin, comprising a foam plastic disc which is formed with an aperture, and the part of the disc surrounding the aperture is covered by a cover foil on one side thereof. A sensor strip is placed below the foil in the aperture and is connected to the stripped end of a plastic insulated lead, whose end portion is covered by the cover foil.

US 2014/316231 A1 discloses an ECG electrode which can be placed within the direct path of x-rays during an imaging scan without inducing an x-ray induced erroneous current. The ECG electrode has a support element with a conductive post on one side electrically connected to a conductive plate on the other side. A dissipative anti-static element in or near the ECG electrode dissipates static electricity which forms on the surfaces of the insulating components in the ECG electrode. The dissipative anti-static element may be, for example, a slightly conductive property of the bulk material used to make the insulating material, or a conductive coating added to the insulating material surfaces. The dissipative anti-static element may also be incorporated in the clamp attached to the conductive post.

WO 2019/057681 A1 relates to signal monitoring leads with dissipative covers and is described with particular application to electrocardiography. In one aspect, a lead set for transferring an electrical biological signal from an electrode to a monitoring device, includes an electrode connector, a monitor connector, a cable, and an electro-static dissipative cover. The electrode connector is configured to electrically connect to an electrode. The monitor connector is configured to electrically connect to a monitor. The first end of the cable is electrically coupled to the electrode connector and a second end electrically coupled to the monitor connector. The static dissipative cover is configured to cover at least one of the electrode connector or the cable.

US 2005/0261565 A1 discloses a well-known type of disc-formed medical electrode having a centred snap connector placed directly over and in direct contact with an electrolyte gel. The centred snap connector projects through an aperture in a foam body. Optionally, a label may be included on the top surface of the foam body. The foam body includes an adhesive backing on its bottom surface. A release liner is adhered to the adhesive backing of the foam body.

However, all the existing prior art electrodes are more or less prone to signal artifacts induced by electrostatic noise and/or signal artifacts caused by mechanical impact on the electrode. Such signal artifacts may be caused by movement of the patient being monitored, movement of clothing, and/or electrostatic charges in the surroundings. These artifacts may complicate visual inspection of the signals by clinicians. In order to reduce this problem, different kinds of software filtering are usually applied to the signals before visual inspection by the clinicians.

Signal artifacts may in particular be problematic in clinical applications such as stress tests, for instance on a bike or running on a treadmill. Misinterpretation of the signals as well as re-recordings due to artefacts in the signal may be a concern. Furthermore, during telemetry monitoring in hospitals, false alarms may be a result of artefacts in the signals. Also during long-term Holter monitoring, that is, ECG monitoring at home doing everyday activities, artefacts in the signals may be a concern.

The object of the present disclosure is to provide a medical electrode having, without the application of any software filtering, an improved signal quality as compared to existing medical electrodes.

In view of this object, a protective disc-formed structure having a lid and an outer rim is arranged at the top side of the plastic foam disc, and the outer rim of the protective disc-formed structure is attached to the medical electrode.

In this way, by attaching the protective disc-formed structure to the medical electrode along the outer rim of the protective disc-formed structure, at least a central part of the medical electrode at the contact medium chamber may be mechanically reinforced by the protective disc-formed structure in such a way that mechanical impact on the sensor strip and/or contact medium in the form of gel disposed in the contact medium chamber may be reduced during application of the electrode on the skin of a patient. Thereby, signal artifacts caused by any kind of touch/direct mechanical impact, such as movement of the patient, surroundings such as clothing, or the electrode itself, may be reduced or eliminated. The already stabilising effect of the weld line may be enhanced by the attachment of the protective disc-formed structure such that a particular strong reinforcement of the medical electrode and in particular the contact medium chamber may be obtained. Deformation of the contact medium chamber could lead to disturbances in the signal.

In an embodiment, the lid of the protective disc-formed structure is made of a plastic material having a tensile modulus being higher than 0.1 GPa and preferably higher than 1 GPa (Test method: ASTM-D-882:18). Thereby, the contact medium chamber may be even better mechanically reinforced by the protective disc-formed structure.

In a structurally particularly advantageous embodiment, the lid of the protective disc-formed structure forms an inner closed chamber when the protective disc-formed structure is attached to the medical electrode. The closed chamber may form a kind of air-cushion in order to dampen or reduce transfer of mechanical impact to the sensor strip and/or contact medium caused by mechanical impacts on the top of the protective disc-formed structure.

In an embodiment, the outer rim of the protective disc-formed structure is attached to the top side of the plastic foil.

In an embodiment, the outer rim of the protective disc-formed structure is attached to the top side of the plastic foil above or radially outside the weld line surrounding the aperture in the plastic foam disc. Thereby, the already stabilising effect of the weld line may be enhanced by the attachment of the outer rim of the protective disc-formed structure and a particular strong reinforcement of the medical electrode may be obtained. Furthermore, the outer rim of the protective disc-formed structure may be attached to the top side of the plastic foil in one operation in which the plastic foil is also being welded to the plastic foam disc, thereby creating the weld line surrounding the aperture in the plastic foam disc.

In an embodiment, a sponge made from a foam material, such as open pore foam or closed cell foam, is arranged between the lid and the top side of the plastic foam disc. Thereby, mechanical impact on the top of the protective disc-formed structure may be even better dampened in that the sponge may create a cushion effect between the lid of the protective disc-formed structure and the top of the contact medium chamber so that mechanical impact on the sensor strip disposed in the contact medium chamber may be further reduced during application of the electrode on the skin of a patient.

In an embodiment, the sponge is arranged between the lid and the top side of the plastic foam disc in a compressed state. Thereby, a slight pressure may be induced on the sensor area of the contact medium chamber and the cushion effect of the sponge may be further enhanced.

In an embodiment, the sponge in its compressed state has a compressed height being less than 50 per cent of an uncompressed height of the sponge before compression and arrangement of the sponge between the lid and the top side of the plastic foam disc. Thereby, the cushion effect of the sponge may be even further enhanced.

In an embodiment, the medical electrode includes an electrostatic shielding covering at least the entire top side of the plastic foam disc, and the electrostatic shielding includes at least an upper dissipative layer and a lower conductive layer. Thereby, the medical electrode may be shielded against electrostatic charges of the surroundings and the signal quality may be improved. By providing an upper dissipative layer, an outer shield is obtained, and by providing a lower conductive layer, any charges are allowed to flow to ground.

In an embodiment, the electrostatic shielding forms a rim area extending from an edge of the plastic foam disc, and, in said rim area, a bottom side of the lower conductive layer of the electrostatic shielding is provided with an electrically conductive and adhesive solid gel layer. Thereby, the lower conductive layer of the electrostatic shielding may be in direct electrical contact with skin of the patient through the electrically conductive and adhesive solid gel layer. Thereby, the lower conductive layer may lead any charges to flow to ground in the form of the skin of the patient.

In an embodiment, the electrostatic shielding is adapted to cover the electric connector. Thereby, also the electric connector may be shielded against electrostatic charges of the surroundings and the signal quality may be further improved. The electric connector is adapted to be electrically connected to not shown monitoring equipment in a well-known way by means of a cable, lead or wire, in that the electric connector has a first snap lock part, for instance a male part, to which a second snap lock part, for instance a female part, of the cable may connect.

A mating connector on a lead/wire and preferably also a first part of the lead/wire may also be covered by the electrostatic shielding. Thereby, the signal quality may be even further improved by reducing electrostatically induced signal disturbances caused at/near the connector and/or wire.

In an embodiment, the electrostatic shielding is provided with at least one flap adapted to be folded about the electric connector and possibly part of a cable connected thereto. Thereby, every side of the electric connector may be shielded against electro-static charges of the surroundings and the signal quality may be even further improved.

In an embodiment, the upper dissipative layer of the electrostatic shielding is oriented polypropylene (OPP), and the lower conductive layer of the electrostatic shielding is preferably formed by electrically conductive ink printed on a lower side of the upper dissipative layer. Preferably, the electrically conductive ink is printed as a grid on a lower side of the upper dissipative layer.

The invention disclosure will now be explained in more detail below by means of examples of embodiments with reference to the very schematic drawing, in which
Fig. 1 is a top view of a prior art medical electrode,
Fig. 2 is a longitudinal cross-sectional view of the prior art medical electrode of Fig. 1,
Fig. 3 is a top view of a medical electrode, according to the present disclosure, provided with protective disc-formed structure,
Fig. 4 is a longitudinal cross-sectional view of the medical electrode of Fig. 3,
Fig. 5 is a top view of a medical electrode, according to the present disclosure, provided with an electrostatic shielding,
Fig. 6 is a longitudinal cross-sectional view of the medical electrode of Fig. 5,
Fig. 7 illustrates a detail of Fig. 6 on a larger scale,
Fig. 8 is a longitudinal cross-sectional view of a medical electrode, according to the present disclosure, provided with a protective disc-formed structure and an electrostatic shielding,
Figs. 9 and 10 illustrate a procedure of applying a medical electrode, according to the present disclosure, with an electrostatic shielding,
Fig. 11 illustrates ECG signals for a prototype medical electrode and a reference medical electrode, respectively, without any noise generating test activity,
Fig. 12 illustrates placement on a torso of a prototype medical electrode and a reference medical electrode, respectively, for testing,
Fig. 13 illustrates ECG signals for a prototype medical electrode provided with an electrostatic shielding and a reference medical electrode, respectively, when shaking a shirt of a test person,
Fig. 14 illustrates ECG signals for a prototype medical electrode provided with an electrostatic shielding and a reference medical electrode, respectively, when tapping on top of the electrodes,
Fig. 15 illustrates ECG signals for a prototype medical electrode provided with a protective disc-formed structure enclosing a sponge and a reference medical electrode, respectively, when tapping on top of the electrodes,
Fig. 16 illustrates ECG signals for a prototype medical electrode provided with a protective disc-formed structure and a reference medical electrode, respectively, when tapping on top of the electrodes (in this figure, the ECG signal for the prototype medical electrodes and the ECG signal for the reference medical electrodes have been illustrated in reverse order as compared with the other figures),
Fig. 17 illustrates ECG signals for a prototype medical electrode provided with an electrostatic shielding and a protective disc-formed structure, and a reference medical electrode, respectively, when shaking a shirt of a test person,
Fig. 18 illustrates ECG signals for a prototype medical electrode provided with an electrostatic shielding and a protective disc-formed structure enclosing a sponge, and a reference medical electrode, respectively, when shaking a shirt of a test person, and
Fig. 19 illustrates ECG signals for a prototype medical electrode provided with an electrostatic shielding and a protective disc-formed structure enclosing a sponge, and a reference medical electrode, respectively, when tapping on top of the electrodes.

In the following, generally, similar elements of different embodiments have been designated by the same reference numerals.

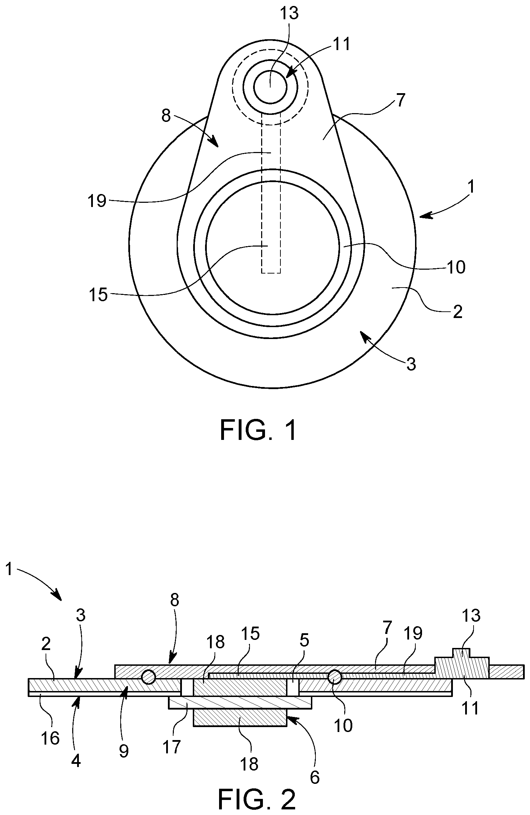

Figs. 1 and 2 illustrate a well-known prior art medical electrode 1 including a plastic foam disc 2 having a top side 3 and a bottom side 4. The plastic foam disc 2 may be more or less circular or may have any other suitable form. The electrode 1 may typically be of the type used for establishing electrical contact between the skin of humans or animals and electrical measuring equipment when performing electrocardiography. The electrode could for instance be a BlueSensor R available from Ambu (Registered Trademark).

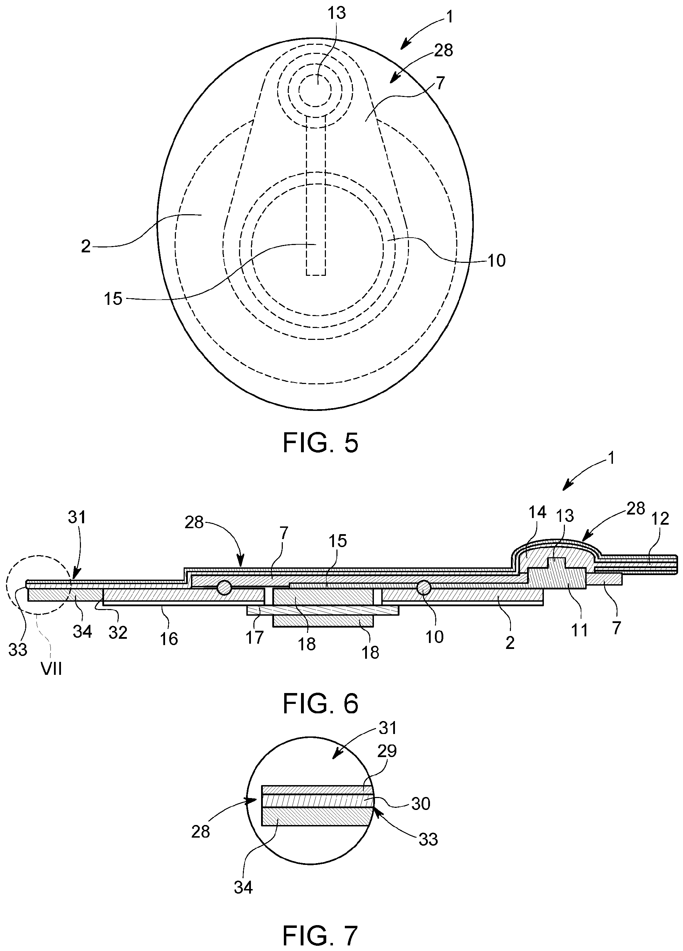

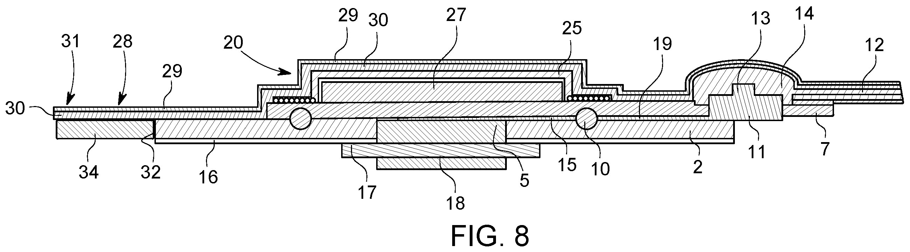

A preferably circular aperture 5 extends at a central part of the plastic foam disc 2 from the top side 3 to the bottom side 4 of the plastic foam disc 2 and thereby forms a contact medium chamber 6. A plastic foil 7 has a top side 8 and a bottom side 9 and is arranged on the top side 3 of the plastic foam disc 2, thereby covering the aperture 5 and at least a part of the surrounding plastic foam disc 2. The bottom side 9 of the plastic foil 7 is welded to the top side 3 of the plastic foam disc 2 along a weld line 10 surrounding the aperture 5. Furthermore, an electric connector 11 is mounted on the plastic foil 7 eccentrically in relation to the central part of the plastic foam disc 2 at which the aperture 5 is arranged. As seen, in the illustrated embodiment, the plastic foil 7 has a teardrop form with the electric connector 11 arranged at a thin end of the teardrop form. The electric connector 11 is adapted to be electrically connected to not shown monitoring equipment in a well-known way by means of a cable 12 in that the electric connector 11 has a first snap lock part 13 to which a second snap lock part 14 of the cable may connect. The second snap lock part 14 and the cable 12 is only illustrated in Figs. 6 and 8. In this embodiment, the first snap lock part 13 is a male connector and the second snap lock part 14 is a female connector. This female connector part 14 on the cable 12 may often be referred to as "cable connector". It may actually also be a crocodile clip. An alternative connector can be an "interface plug" is arranged on the male stud on the electrode (or integrated instead of the male stud) and is adapted to receive a banana plug. Alternatively, the cable or wire can be integrated into the electrode, e.g. as in Ambu^{®} BlueSensor QR.

A sensor strip 15 is disposed in the contact medium chamber 6 and is electrically connected to the electric connector 11 by means of an extension 19 of the sensor strip 15 or possibly by means of a dedicated conductive element. The sensor strip 15 may for instance have the form of a metal strip, for instance a silver chloride coated silver strip, a carbon strip, or a sensor area printed by means of conductive ink on a strip. A layer 16 of pressure-sensitive adhesive is applied to the bottom side 4 of the plastic foam disc 2.

A sponge 17 is arranged across the contact medium chamber 6, e.g. by a peripheral edge of the sponge 17 being adhered to the bottom side 4 of the plastic foam disc 2 by means of the layer 16 of pressure-sensitive adhesive. The necessary electrical contact between the sensor strip 15 and the skin of a person can be established by means of a contact medium 18 which has been at least partly absorbed in the sponge 17. The contact medium 18 may generally be a paste-like electrolyte. The contact medium 18 is typically a conductive (wet) gel. The conductive gel typically comprises ions, such as chloride ions ( CI⁻) that transfer bioelectric signals from the patient's skin to the sensor strip 15. Preferably, the gel is a NaCl + KCI containing gel. The layer 16 of pressure-sensitive adhesive applied to the bottom side 4 of the plastic foam disc 2 may ensure that the medical electrode 1 is securely adhered to the skin of a person during use.

As patients are often to be monitored for long periods of time, e. g. several days, it is important that the medical electrode 1 adds as little to the discomfort of the patient or carrier as possible. This is provided for to a great extent by the use of the plastic foam disc 2, which is a soft and pliable material. The preferred foam plastics for the plastic foam disc 2 is PVC foam; however, other materials may be used, such as polyolefines, natural or synthetic rubber, biopolymers, polyesters, polyethers, polyurethanes, polyamides, or copolymers such as ABS or mixtures thereof or laminates comprising one or more of these may further be used. It is also important that the gel-like contact medium 18, which is generally used for establishing good electrical connection between the sensor strip 15 and the skin, can be kept intact during the entire period of operation. This means that the walls defining the contact medium chamber 6 must be so tight as to allow no considerable diffusion of the constituent components of the contact medium 18.

Although in Figs. 1 and 2, as explained above, it has been illustrated that the plastic foil 7 is welded to the top side 3 of the plastic foam disc 2 along a weld line 10 surrounding the aperture 5, in reality, the weld line 10 may be broader than illustrated and may extend to the edge of the contact medium chamber 6 as it will be explained in the following. The preferred material for the plastic foil 7 which in the case of the Ambu BlueSensor is in blue colour, is polyvinylChloride (PVC); however, other materials may be used, such as polyolefines, natural or synthetic rubber, biopolymers, polyesters, polyethers, polyurethanes, polyamides or copolymers such as ABS or mixtures thereof or laminates comprising one or more of these may also be used.

The prior art medical electrode 1 illustrated in Figs. 1 and 2 may be manufactured by placing the various parts in their proper mutual positions between a not shown flat metal substrate and a not shown metal pressing plate, and whereby the pressing plate is subsequently moved so far down towards the substrate that the part of the plastic foam disc 2 aligned with the plastic foil 7 is somewhat compressed. The substrate and the pressing plate may form a capacitor inserted in a high-frequency circuit (not shown), and heat may be developed between these plates as a consequence of dielectric losses. The heat causes walls of compressed plastic foam cells to fuse partly so that the part of the plastic foam disc 2 in question is fixed in the compressed shape. At the same time the plastic foil 7 is welded to the plastic foam disc 2. The extension 19 of the sensor strip 15 extends through the welding formed to the electric connector 11. Other suitable manufacturing methods are, of course, also possible.

The compression of the part of the plastic foam disc 2 surrounding the aperture 5 causes the aperture to become so shallow that the necessary electrical contact between the sensor strip 15 and the skin can easily be established by means of the sponge 17, in which the contact medium 18 has been absorbed and which is adhered to the plastic foam disc 2 around the aperture 5.

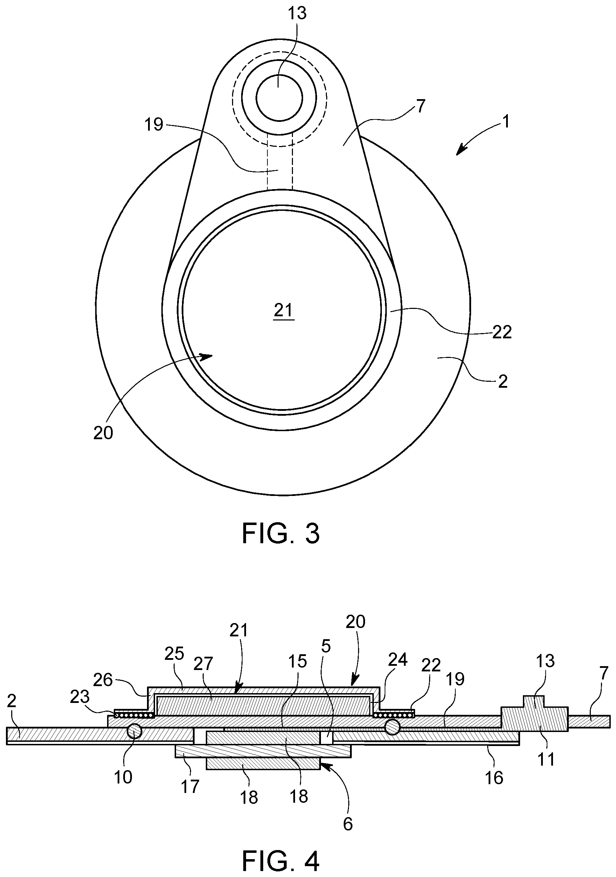

Figs. 3 and 4 illustrate a medical electrode 1 according to the present disclosure wherein a protective disc-formed structure 20 having a lid 21 and an outer rim 22 is arranged at the top side 3 of the plastic foam disc 2. The outer rim 22 of the disc-formed structure is attached to the underlying medical electrode 1, preferably by being attached to the top side 8 of the plastic foil 7, and preferably by adhesion, welding or hot melting. Adhesion may for instance by performed by means of sticky double-sided tape 23 arranged between the outer rim 22 and the top side 8 of the plastic foil 7. Thereby, at least a central part of the medical electrode 1 at the contact medium chamber 6 may be mechanically reinforced by the protective disc-formed structure 20 in such a way that mechanical impact on the sensor strip 15 disposed in the contact medium chamber 6 may be reduced during application of the medical electrode on the skin of a patient. Further, the presence of the lid 21 in combination with weld line 10 that surrounds the contact medium chamber 6 may provide stability against deformation of the contact medium chamber that could induce mechanical artifacts in the biosignals detected by the electrode. Thereby, signal artifacts caused by any kind of mechanical impact, such as movement of the patient, surroundings such as clothing, or the medical electrode itself, may be reduced or eliminated. As a further advantage, the addition of the protective disc-formed structure 20 also strengthens the medical electrode 1 in such a way that it may be avoided that the contact medium 18, arranged in the contact medium chamber 6 and at least partly absorbed in the sponge 17, inadvertently is pressed out of its location when handling the medical electrode.

Preferably, the lid 21 of the protective disc-formed structure 20 is made of a plastic material having a tensile modulus being higher than 0.1 GPa and preferably higher than 1 GPa. The material of the protective disc-formed structure 20 may be a combination film in the form of A-PET / LDPE laminate or film or other alternative stiff materials such as PE, PP, ABS, PET or laminates comprising one or more layers thereof. The protective disc-formed structure 20 may have a thickness of 100-400 µm, preferably 200-300 µm, for instance 250 µm.

As seen, the lid 21 of the protective disc-formed structure 20 forms an inner closed chamber 24 when the protective disc-formed structure 20 is attached to the medical electrode 1. In the illustrated embodiment, the lid 21 is formed by a top wall 25 and a side wall 26 connecting the top wall 25 and the outer rim 22. The inner closed chamber 24 may form a kind of air-cushion in order to prevent or at least reduce transfer of mechanical impact from the top of the protective disc-formed structure 20 to the contact medium chamber 6 and the sensor strip 15. Such mechanical impact on the contact medium chamber 6 could otherwise induce artefacts in the biosignals obtained from the medical electrode.

In the illustrated embodiment, the outer rim 22 of the protective disc-formed structure 20 is attached to the top side 8 of the plastic foil 7 above the weld line 10 surrounding the aperture 5 in the plastic foam disc 2. However, the outer rim 22 of the protective disc-formed structure 20 may just as well be attached to the top side 8 of the plastic foil 7 slightly radially outside the weld line 10 surrounding the aperture 5 in the plastic foam disc 2. These embodiments are preferred due to enhanced stability of the medical electrode 1 against general deforming. The already stabilising effect of the weld line 10 may be enhanced by the attachment of the outer rim 22 of the protective disc-formed structure 20 such that a synergistic effect may be obtained and thereby a particular strong reinforcement of the contact medium chamber 6 and the medical electrode 1 may be obtained against mechanical impact. In the case that the protective disc-formed structure 20 is attached to the plastic foil 7 by means of welding, the welding process may be performed in one welding step whereby the three components, the protective disc-formed structure 20, the plastic foil 7 and the plastic foam disc 2 are connected together.

As further illustrated in Fig. 4, a sponge 27 made from a foam material, such as open pore foam or closed cell foam, may be arranged between the lid 21 and the top side 3 of the plastic foam disc 2. Furthermore, in the illustrated embodiment, the sponge 27 is arranged between the lid 21 and the top side 8 of the plastic foil 7. Thereby, mechanical impact on the top of the protective disc-formed structure 20 may be even better dampened in that the sponge 27 may create a cushion effect between the lid 21 of the protective disc-formed structure 20 and the top of the contact medium chamber 6 so that mechanical impact on the sensor strip 15 disposed in the contact medium chamber 6 may be further reduced during application of the medical electrode 1 on the skin of a patient.

The sponge 27 is arranged between the lid 21 and the top side of the plastic foam disc 2 in a compressed state. Thereby, a slight pressure may be induced on a sensor area of the contact medium chamber 6 and the cushion effect of the sponge 27 may be further enhanced. Preferably, the sponge 27 in its compressed state has a compressed height being less than 50 per cent of an uncompressed height of the sponge 27 before compression and arrangement of the sponge 27 between the lid 21 and the top side 3 of the plastic foam disc 2.

The sponge 27 may for instance be formed of a polyurethane or polyether foam, such as polyurethane 017H commercially available from Bramming Plast-Industri A/S, Denmark. The thickness may for instance be more than 5mm for a lid 21 with a height of about 2 mm, the density may for instance be about 15 kg/m³(ISO 845), a compression hardness may for instance be about 2.5 kPa (ISO 3386 (40%)) and a tensile strength may for instance be about 90 kPa (ISO 1798).

Figs. 5 and 6 illustrate a medical electrode 1 according to the present disclosure wherein the medical electrode 1 includes an electrostatic shielding 28 in the form of a sheet covering the entire medical electrode 1. The electrostatic shielding 28 includes at least an upper dissipative layer 29 and a lower conductive layer 30. Thereby, the medical electrode 1 may be shielded against electrostatic charges of the surroundings and the signal quality may be improved. By providing an upper dissipative layer 29 , an outer shield is obtained, and by providing a lower conductive layer 30, any charges are allowed to flow to ground. A not shown adhesive material, such as acrylic, silicone etc., may be attached to the lower side of the lower conductive layer 30 in order to adhere the electrostatic shielding 28 to the medical electrode 1. In the embodiment of Figs. 5 and 6, the medical electrode 1 has not been provided with a protective disc-formed structure 20 as illustrated in the embodiment of Figs. 3 and 4.

The embodiment disclosed in Figs. 5 and 6 is defined as a medical electrode 1 including a plastic foam disc 2 having a top side 3 and a bottom side 4. Furthermore, an aperture 5 extends at a central part of the plastic foam disc from the top side to the bottom side and thereby forms a contact medium chamber 6. A plastic foil 7 has a top side 8 and a bottom side 9 and is arranged on the top side of the plastic foam disc, thereby covering the aperture and at least a part of the surrounding plastic foam disc. The bottom side of the plastic foil is welded to the top side of the plastic foam disc along a weld line 10 surrounding the aperture. An electric connector 11 is mounted on the plastic foil eccentrically in relation to the central part of the plastic foam disc. The electric connector is adapted to be electrically connected to monitoring equipment. A sensor strip 15 is disposed in the contact medium chamber and is electrically connected to the electric connector. A layer of pressure-sensitive adhesive 16 is applied to the bottom side of the plastic foam disc. Furthermore, the medical electrode includes an electrostatic shielding 28 covering at least the entire top side of the plastic foam disc, and the electrostatic shielding includes at least an upper dissipative layer 29 and a lower conductive layer 30. Preferably, in this embodiment, the upper dissipative layer of the electrostatic shielding is oriented polypropylene (OPP), and preferably, the lower conductive layer of the electrostatic shielding is formed by electrically conductive ink printed as a grid on a lower side of the upper dissipative layer.

As seen, the electrostatic shielding 28 forms a rim area 31 extending from an edge 32 of the plastic foam disc 2, and in said rim area 31, a bottom side 33 of the lower conductive layer 30 of the electrostatic shielding 28 is provided with an electrically conductive and adhesive solid gel layer 34. Thereby, the lower conductive layer 30 of the electrostatic shielding 28 may be in direct electrical contact with skin of the patient through the electrically conductive and adhesive solid gel layer 34. Thereby, the lower conductive layer 30 may lead any charges to flow to the skin of the patient, thereby acting as ground connection. The electrically conductive and adhesive solid gel layer 34 may for instance be of the type AG625 SENSING GEL commercially available from Axelgaard Manufacturing CO., LTD., USA.

Fig. 8 illustrates an embodiment of the medical electrode 1 according to the present disclosure corresponding to the embodiment illustrated in Figs. 5 and 6, however, in the embodiment of Fig. 8, the medical electrode 1 includes an electrostatic shielding 28 covering the entire medical electrode 1 including a protective disc-formed structure 20 including optionally a sponge 27 as described above. The protective disc-formed structure 20 provides further distance between the remaining part of the medical electrode 1 and the electrostatic shielding 28 which under certain circumstances may improve the electrostatic shielding of the medical electrode 1. It is noted, however, that in an alternative embodiment, the protective disc-formed structure 20 including the sponge 27 or without the sponge 27 may be arranged on the top side of the electrostatic shielding 28 so that the protective disc-formed structure 20 and the sponge 27 are not covered by the electrostatic shielding 28. This may not alter the electrostatic shielding function provided by the electrostatic shielding 28, but possibly, it may facilitate production of the medical electrode 1.

As seen in the embodiments of Figs. 5, 6 and 8, the electrostatic shielding 28 may also be adapted to cover the electric connector 11. Thereby, also the electric connector 11 may be shielded against electrostatic charges of the surroundings and the signal quality may be further improved. As mentioned above, the electric connector 11 is adapted to be electrically connected to not shown monitoring equipment in a well-known way by means of a cable 12. The electric connector 11 has a first snap lock part 13 to which a second snap lock part 14 of the cable may connect. According to the embodiments of Figs. 5, 6 and 8, both the first snap lock part 13 and the second snap lock part 14 and at least a part of the cable 12 may be covered by the electrostatic shielding 28. Preferably, the cable 12 is a shielded wire, such as a coax wire.

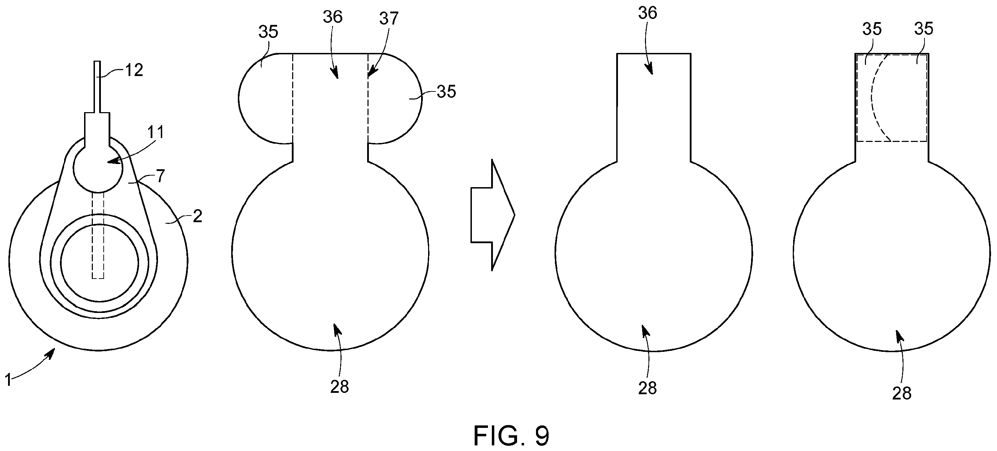

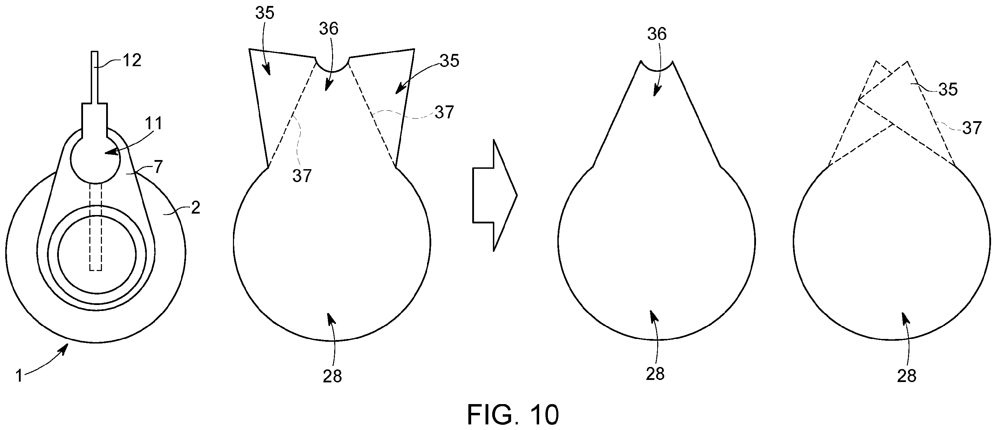

Figs. 9 and 10 illustrate two different embodiments in which the sheet forming the electrostatic shielding 28 is provided with two flaps 35 adapted to be folded about the electric connector 11. Each flaps 35 is arranged bendable in relation to a midportion 36 of the electrostatic shielding 28. In Fig. 10 optional bending lines 37 between the midportion 36 and the respective flaps 35 are illustrated. Alternatively, only one flap may be provided which may be bent one or two times in order to appropriately cover all sides of the electric connector 11. For instance, the one or two flaps 35 adapted to be folded about the electric connector 11 may be provided with one or more not shown separate release liner(s) to be removed before folding the flap or flaps about the electric connector 11. The foldable electrostatic shielding may be applicable to cover the cable 12 and connector 11 irrespective of the connector type employed. Different connector types are discussed above. If the cable 12 is pre-attached to the medical electrode (no connector), then the electrostatic shielding 28 may be bent around the cable at the factory.

Preferably, generally, the upper dissipative layer 29 of the electrostatic shielding 28 is oriented polypropylene (OPP), and preferably, the lower conductive layer 30 of the electrostatic shielding is formed by electrically conductive ink printed on a lower side of the upper dissipative layer. However, other suitable materials may also be employed for the upper dissipative layer 29 of the electrostatic shielding, such as for instance polypropylene in general or polyethylene in general. Preferably, the electrically conductive ink is printed as a grid on the lower side of the upper dissipative layer. Such electrostatic shielding is commercially available, for instance in the form of "ESD Anti-static Grid Tapes" from Conrad Electronic SE, Germany. The upper dissipative layer 29 of the electrostatic shielding 28 may for instance have a surface resistance of 10⁴ Ohm to 10¹⁰ Ohm, such as about 10⁹ Ohm, and the lower conductive layer 30 may for instance have a resistivity of about 1000 Ohm·m.

In order to demonstrate the effect of the different embodiments of the medical electrode 1 according to the present disclosure, a number of tests have been performed as described in the following.

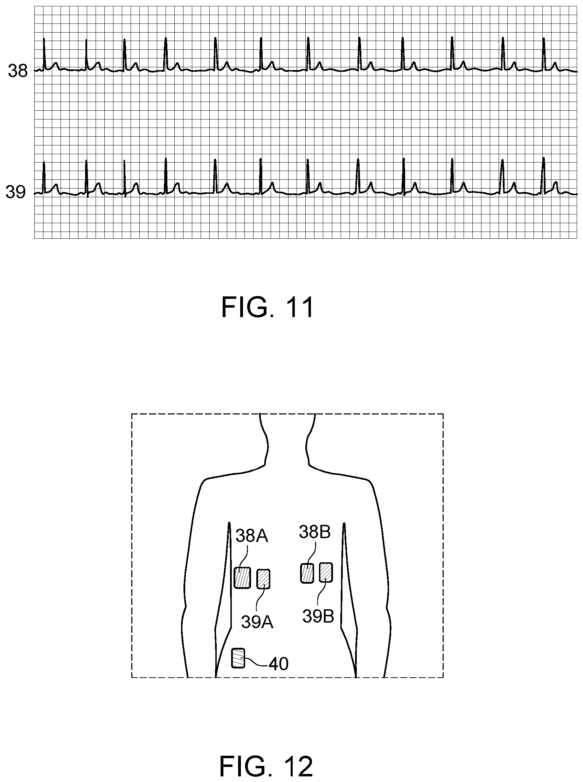

Fig. 12 illustrates the test setup, whereby for each test, prototype medical electrodes 38A, 38B according to the present disclosure as well as reference prior art medical electrodes 39A, 39B and a single neutral (ground) medical electrode 40 are placed as illustrated in the figure on a torso of a test person. In each case, the reference prior art medical electrodes 39A, 39B are of the type Ambu BlueSensor. The ECG recordings were obtained using AMEDTEC ECGpro CardioPart and their standard software adapted to be able to record bipolar signals. No pre-defined software filters were applied during recording. The signals were recorded with two sets of medical electrodes, so the prototype signal and the reference signal could be recorded simultaneously. Importantly, the amplitudes of the ECG signals were similar on both leads. If not the case, the prototypes were replaced and repositioned until the amplitudes were substantially similar as on the ECG signal illustrated in Fig. 11.

According to the test protocol, the following activities were performed simultaneously on the four test electrodes:
1) Tapping on top of electrodes in order to induce mechanical impact
2) Shaking shirt in order to induce electrostatic disturbances

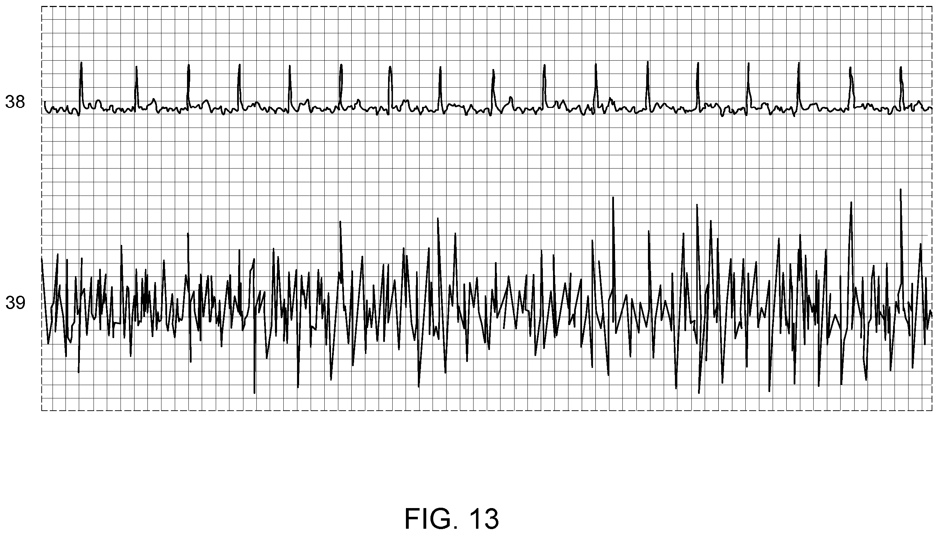

A selection of the test results are illustrated in Figs. 13 to 19 as follows. In each test performed, it has been seen that the signal of the prototype medical electrodes 38A, 38B represents a substantive reduction of the signal artefacts which are present in the signal of the reference medical electrodes 39A, 39B.

Fig. 13 illustrates ECG signals for prototype medical electrodes 38A, 38B provided with an electrostatic shielding 28 (similar to medical electrode shown in Fig. 6) and reference medical electrodes 39A, 39B, respectively, when shaking a shirt of a test person in order to induce electrostatic disturbances.

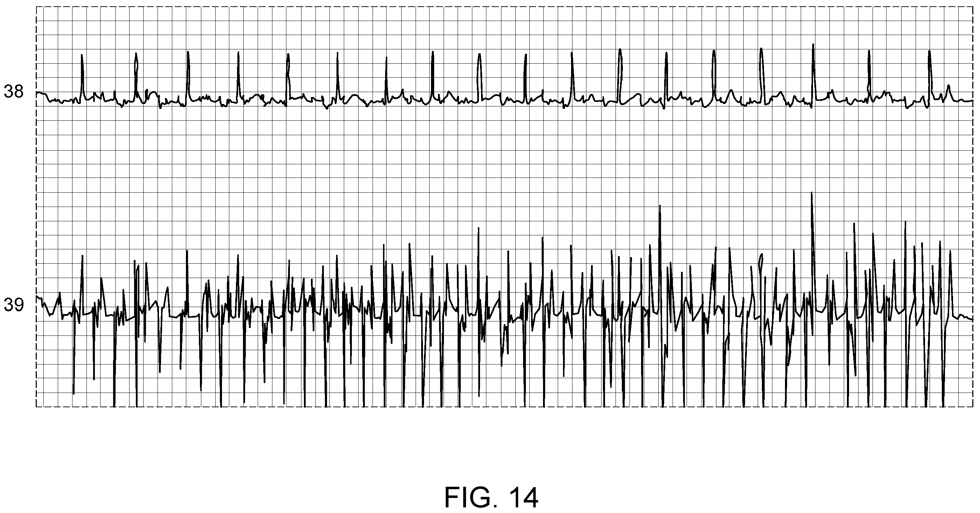

Fig. 14 illustrates ECG signals for prototype medical electrodes 38A, 38B provided with an electrostatic shielding 28 (similar to medical electrode shown in Fig. 6) and reference medical electrodes 39A, 39B, respectively, when tapping on top of the electrodes.

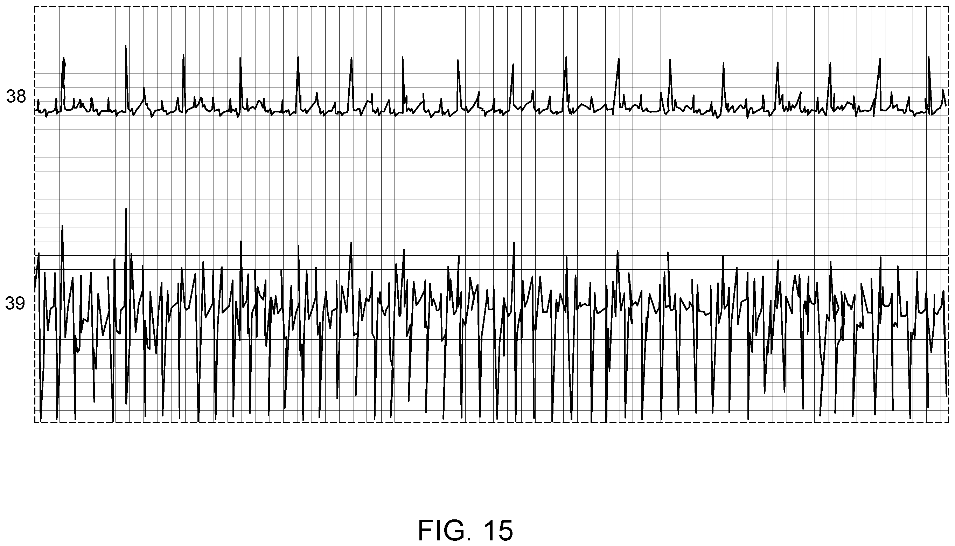

Fig. 15 illustrates ECG signals for prototype medical electrodes 38A, 38B provided with a protective disc-formed structure 20 enclosing a sponge 27 (similar to medical electrode shown in Figs. 3 and 4) and reference medical electrodes 39A, 39B, respectively, when tapping on top of the electrodes.

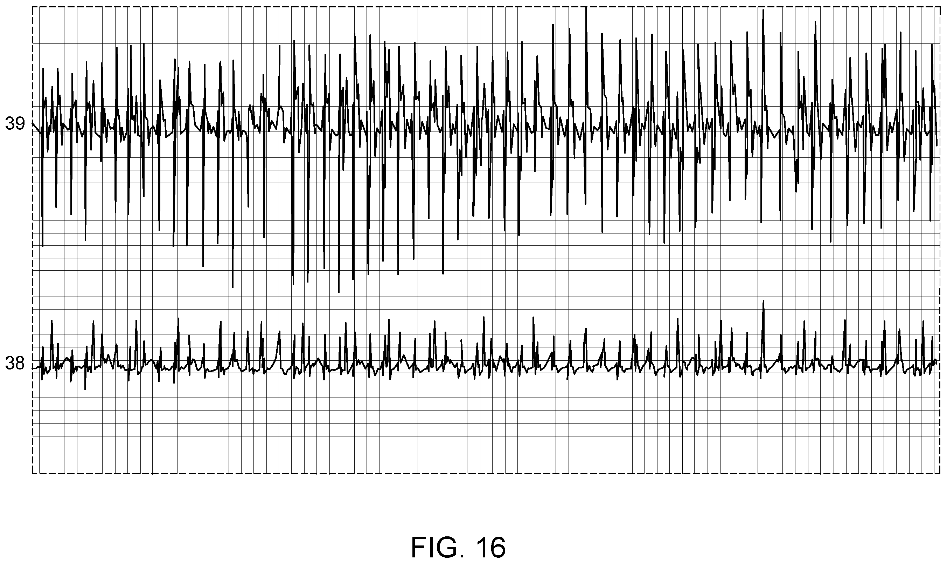

Fig. 16 illustrates ECG signals for prototype medical electrodes 38A, 38B provided with a protective disc-formed structure 20 (similar to medical electrode shown in Figs. 3 and 4, but without sponge 27) and reference medical electrodes 39A, 39B, respectively, when tapping on top of the electrodes. In is noted that in the diagram of Fig. 16, the ECG signal for the prototype medical electrodes 38A, 38B is shown *below* the ECG signal for the reference medical electrodes 39A, 39B, as opposed to the corresponding diagrams illustrated in the other figures.

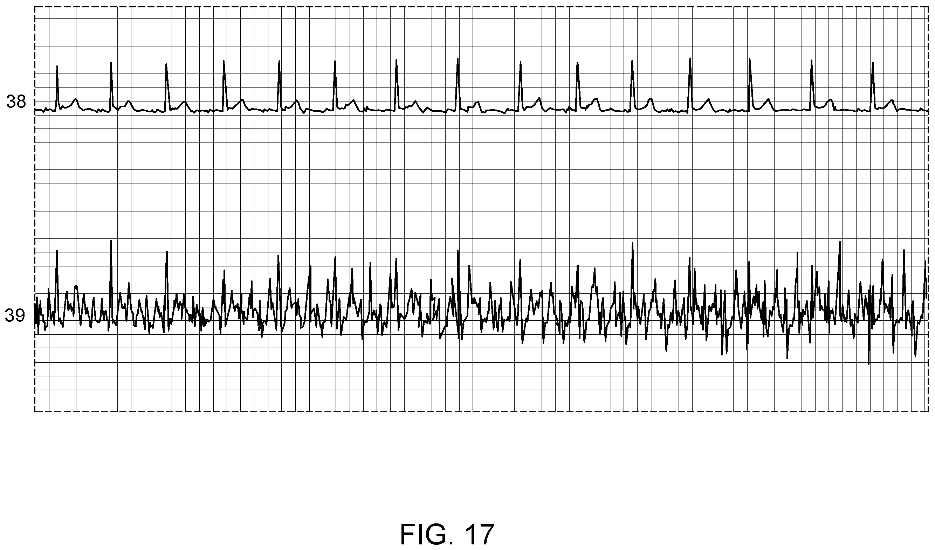

Fig. 17 illustrates ECG signals for prototype medical electrodes 38A, 38B provided with an electrostatic shielding 28 (similar to medical electrode shown in Fig. 8, but without sponge 27) and a protective disc-formed structure 20, and reference medical electrodes 39A, 39B, respectively, when shaking a shirt of a test person.

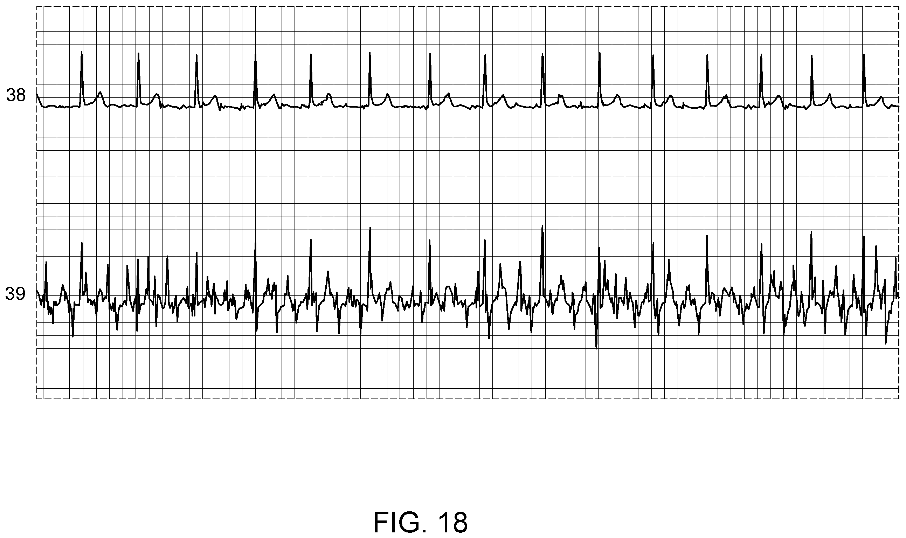

Fig. 18 illustrates ECG signals for prototype medical electrodes 38A, 38B provided with an electrostatic shielding 28 and a protective disc-formed structure 20 enclosing a sponge 27 (similar to medical electrode shown in Fig. 8), and reference medical electrodes 39A, 39B, respectively, when shaking a shirt of a test person.

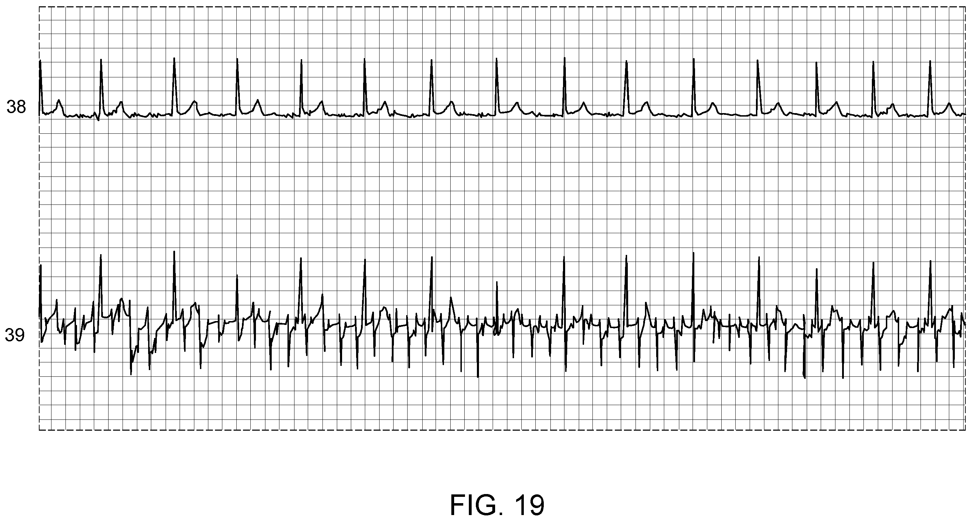

Fig. 19 illustrates ECG signals for a prototype medical electrode 38A, 38B provided with an electrostatic shielding 28 and a protective disc-formed structure 20 enclosing a sponge 27 (similar to medical electrode shown in Fig. 8), and a reference medical electrode 39A, 39B, respectively, when tapping on top of the electrodes.

### List of reference numbers

- 1: medical electrode
- 2: plastic foam disc
- 3: top side of plastic foam disc
- 4: bottom side of plastic foam disc
- 5: aperture forming contact medium chamber
- 6: contact medium chamber
- 7: plastic foil
- 8: top side of plastic foil
- 9: bottom side of plastic foil
- 10: weld line surrounding aperture
- 11: electric connector mounted on plastic foil
- 12: cable
- 13: first snap lock part
- 14: second snap lock part
- 15: sensor strip
- 16: layer of pressure-sensitive adhesive
- 17: sponge
- 18: contact medium
- 19: extension of sensor strip
- 20: protective disc-formed structure
- 21: lid of protective disc-formed structure
- 22: outer rim of protective disc-formed structure
- 23: sticky double-sided tape
- 24: inner closed chamber of lid
- 25: top wall of lid
- 26: side wall of lid
- 27: sponge
- 28: electrostatic shielding
- 29: upper dissipative layer of electrostatic shielding
- 30: lower conductive layer of electrostatic shielding
- 31: rim area of electrostatic shielding
- 32: edge of plastic foam disc
- 33: bottom side of lower conductive layer
- 34: electrically conductive and adhesive solid gel layer
- 35: flap of electrostatic shielding
- 36: folding line between flap and midportion
- 37: midportion of electrostatic shielding
- 38A, 38B: prototype medical electrode
- 39A, 39B: reference prior art medical electrode
- 40: neutral (ground) medical electrode

## Claims

1. A medical electrode (1) including a plastic foam disc (2) having a top side (3) and a bottom side (4), an aperture (5) extending at a central part of the plastic foam disc (2) from the top side (3) to the bottom side (4) and thereby forming a contact medium chamber (6), a plastic foil (7) having a top side (8) and a bottom side (9) and being arranged on the top side (3) of the plastic foam disc (2), thereby covering the aperture (5) and at least a part of the surrounding plastic foam disc (2), the plastic foil (7) being welded to the plastic foam disc (2) along a weld line (10) surrounding the aperture (5), an electric connector (11) being mounted on the plastic foil (7) eccentrically in relation to the central part of the plastic foam disc (2), the electric connector (11) being adapted to be electrically connected to monitoring equipment, a sensor strip (15) disposed in the contact medium chamber (6) and electrically connected to the electric connector (11), and a layer of pressure-sensitive adhesive (16) applied to the bottom side (4) of the plastic foam disc (2), wherein a protective disc-formed structure (20) has a lid (21) and an outer rim (22) and is arranged at the top side (3) of the plastic foam disc (2), and wherein the outer rim (22) of the protective disc-formed structure (20) is attached to the medical electrode (1).

2. A medical electrode according to claim 1, wherein the lid (21) of the protective disc-formed structure (20) is made of a plastic material having a tensile modulus being higher than 0.1 GPa and preferably higher than 1 GPa.

3. A medical electrode according to claim 1 or 2, wherein the lid (21) of the protective disc-formed structure (20) forms an inner closed chamber (24) when the protective disc-formed structure (20) is attached to the medical electrode (1).

4. A medical electrode according to any one of the preceding claims, wherein the outer rim (22) of the protective disc-formed structure (20) is attached to the top side (8) of the plastic foil (7).

5. A medical electrode according to claim 4, wherein the outer rim (22) of the protective disc-formed structure (20) is attached to the top side (8) of the plastic foil (7) above or radially outside the weld line (10) surrounding the aperture (5) in the plastic foam disc (2).

6. A medical electrode according to any one of the preceding claims, wherein a sponge (17) made from a foam material, such as open pore foam or closed cell foam, is arranged between the lid (21) and the top side (3) of the plastic foam disc (2) and/or the plastic foil (7).

7. A medical electrode according to claim 6, wherein the sponge (17) is arranged between the lid (21) and the top side (3) of the plastic foam disc (2) in a compressed state.

8. A medical electrode according to claim 7, wherein the sponge (17) in its compressed state has a compressed height being less than 50 per cent of an uncompressed height of the sponge (17) before compression and arrangement of the sponge (17) between the lid (21) and the top side (3) of the plastic foam disc (2) and/or the plastic foil (7).

9. A medical electrode according to any one of the preceding claims, wherein the medical electrode (1) includes an electrostatic shielding (28) covering at least the entire top side (3) of the plastic foam disc (2), and wherein the electrostatic shielding (28) includes at least an upper dissipative layer (29) and a lower conductive layer (30).

10. A medical electrode according to claim 9, wherein the electrostatic shielding (28) forms a rim area (31) extending from an edge (32) of the plastic foam disc (2), and wherein, in said rim area (31), a bottom side (33) of the lower conductive layer (30) of the electrostatic shielding (28) is provided with an electrically conductive and adhesive solid gel layer (34).

11. A medical electrode according to claim 9 or 10, wherein the electrostatic shielding (28) is adapted to cover the electric connector (11).

12. A medical electrode according to claim 11, wherein the electrostatic shielding (28) is provided with at least one flap (35) adapted to be folded about the electric connector (11) and possibly part of a cable (12) connected thereto.

13. A medical electrode according to any one of the claims 9 to 12, wherein the upper dissipative layer (29) of the electrostatic shielding (28) is oriented polypropylene (OPP), and wherein the lower conductive layer (30) of the electrostatic shielding (28) is formed by electrically conductive ink printed on a lower side of the upper dissipative layer (29).

## Patentansprüche

1. Medizinische Elektrode (1), die eine Kunststoffschaumscheibe (2) mit einer Oberseite (3) und einer Unterseite (4), eine Öffnung (5), die sich in einem zentralen Teil der Kunststoffschaumscheibe (2) von der Oberseite (3) zur Unterseite (4) erstreckt und dadurch eine Kontaktmittelkammer (6) bildet, und eine Kunststofffolie (7) mit einer Oberseite (8) und einer Unterseite (9), die auf der Oberseite (3) der Kunststoffschaumscheibe (2) angeordnet ist und dadurch die Öffnung (5) und mindestens einen Teil der umgebenden Kunststoffschaumscheibe (2) abdeckt, beinhaltet, wobei die Kunststofffolie (7) entlang einer Schweißlinie (10), die die Öffnung (5) umgibt, mit der Kunststoffschaumscheibe (2) verschweißt ist, wobei ein elektrischer Anschluss (11) exzentrisch zum mittleren Teil der Kunststoffschaumscheibe (2) auf der Kunststofffolie (7) angebracht ist, wobei der elektrische Anschluss (11) so ausgelegt ist, dass er elektrisch mit einem Überwachungsgerät verbunden werden kann, wobei ein Sensorstreifen (15) in der Kontaktmittelkammer (6) angeordnet und elektrisch mit dem elektrischen Anschluss (11) verbunden ist, und eine Schicht aus druckempfindlichem Klebstoff (16) auf der Unterseite (4) der Kunststoffschaumscheibe (2) angebracht ist, wobei eine scheibenförmigen Schutzstruktur (20) einen Deckel (21) und einen äußeren Rand (22) aufweist und an der Oberseite (3) der Kunststoffschaumscheibe (2) angeordnet ist, und wobei der äußere Rand (22) der scheibenförmigen Schutzstruktur (20) an der medizinischen Elektrode (1) befestigt ist.

2. Medizinische Elektrode nach Anspruch 1, wobei der Deckel (21) der scheibenförmigen Schutzstruktur (20) aus einem Kunststoffmaterial mit einem Zugmodul von mehr als 0,1 GPa und vorzugsweise mehr als 1 GPa hergestellt ist.

3. Medizinische Elektrode nach Anspruch 1 oder 2, wobei der Deckel (21) der scheibenförmigen Schutzstruktur (20) eine geschlossene Innenkammer (24) bildet, wenn die scheibenförmige Schutzstruktur (20) an der medizinischen Elektrode (1) befestigt ist.

4. Medizinische Elektrode nach einem der vorstehenden Ansprüche, wobei der äußere Rand (22) der scheibenförmigen Schutzstruktur (20) an der Oberseite (8) der Kunststofffolie (7) befestigt ist.

5. Medizinische Elektrode nach Anspruch 4, wobei der äußere Rand (22) der scheibenförmigen Schutzstruktur (20) an der Oberseite (8) der Kunststofffolie (7) oberhalb oder radial außerhalb der Schweißlinie (10), die die Öffnung (5) in der Kunststoffschaumscheibe (2) umgibt, befestigt ist.

6. Medizinische Elektrode nach einem der vorstehenden Ansprüche, wobei zwischen dem Deckel (21) und der Oberseite (3) der Kunststoffschaumscheibe (2) und/oder der Kunststofffolie (7) ein Schwamm (17) aus einem Schaumstoff, wie z. B. einem offenporigen oder geschlossenzelligen Schaumstoff, angeordnet ist.

7. Medizinische Elektrode nach Anspruch 6, wobei der Schwamm (17) in einem komprimiertem Zustand zwischen dem Deckel (21) und der Oberseite (3) der Kunststoffschaumscheibe (2) angeordnet ist.

8. Medizinische Elektrode nach Anspruch 7, wobei der Schwamm (17) in seinem komprimierten Zustand eine komprimierte Höhe von weniger als 50 Prozent einer unkomprimierten Höhe des Schwamms (17) vor Komprimieren und Anordnung des Schwamms (17) zwischen dem Deckel (21) und der Oberseite (3) der Kunststoffschaumscheibe (2) und/oder der Kunststofffolie (7) aufweist.

9. Medizinische Elektrode nach einem der vorstehenden Ansprüche, wobei die medizinische Elektrode (1) eine elektrostatische Abschirmung (28) beinhaltet, die mindestens die gesamte Oberseite (3) der Kunststoffschaumscheibe (2) bedeckt, und wobei die elektrostatische Abschirmung (28) mindestens eine obere dissipative Schicht (29) und eine untere leitfähige Schicht (30) beinhaltet.

10. Medizinische Elektrode nach Anspruch 9, wobei die elektrostatische Abschirmung (28) einen Randbereich (31) bildet, der sich von einem Rand (32) der Kunststoffschaumscheibe (2) aus erstreckt, und wobei in dem Randbereich (31) eine Unterseite (33) der unteren leitfähigen Schicht (30) der elektrostatischen Abschirmung (28) mit einer elektrisch leitfähigen und klebenden festen Gelschicht (34) versehen ist.

11. Medizinische Elektrode nach Anspruch 9 oder 10, wobei die elektrostatische Abschirmung (28) so ausgelegt ist, dass sie den elektrischen Anschluss (11) abdeckt.

12. Medizinische Elektrode nach Anspruch 11, wobei die elektrostatische Abschirmung (28) mit mindestens einer Klappe (35) versehen ist, die um den elektrischen Verbinder (11) und möglicherweise einen Teil eines damit verbundenen Kabels (12) gefaltet werden kann.

13. Medizinische Elektrode nach einem der Ansprüche 9 bis 12, wobei die obere dissipative Schicht (29) der elektrostatischen Abschirmung (28) aus orientiertem Polypropylen (OPP) besteht und wobei die untere leitfähige Schicht (30) der elektrostatischen Abschirmung (28) durch elektrisch leitfähige Tinte gebildet wird, die auf eine Unterseite der oberen dissipativen Schicht (29) aufgedruckt ist.

## Revendications

1. Électrode médicale (1) incluant un disque en mousse plastique (2) présentant un côté supérieur (3) et un côté inférieur (4), une ouverture (5) s'étendant à une partie centrale du disque en mousse plastique (2) du côté supérieur (3) au côté inférieur (4) et formant ainsi une chambre de milieu de contact (6), une feuille plastique (7) présentant un côté supérieur (8) et un côté inférieur (9) et étant agencée sur le côté supérieur (3) du disque en mousse plastique (2), couvrant ainsi l'ouverture (5) et au moins une partie du disque en mousse plastique (2) environnant, la feuille plastique (7) étant soudée au disque en mousse plastique (2) le long d'une ligne de soudure (10) entourant l'ouverture (5), un connecteur électrique (11) étant monté sur la feuille plastique (7) de manière excentrique par rapport à la partie centrale du disque en mousse plastique (2), le connecteur électrique (11) étant adapté pour être connecté électriquement à un équipement de surveillance, une bande de capteurs (15) étant disposée dans la chambre de milieu de contact (6) et connectée électriquement au connecteur électrique (11), et une couche d'adhésif sensible à la pression (16) étant appliquée sur le côté inférieur (4) du disque en mousse plastique (2), dans laquelle une structure protectrice en forme de disque (20) présente un couvercle (21) et un bord extérieur (22) et est agencée sur le côté supérieur (3) du disque en mousse plastique (2), et dans laquelle le bord extérieur (22) de la structure protectrice en forme de disque (20) est fixé à l'électrode médicale (1).

2. Électrode médicale selon la revendication 1, dans laquelle le couvercle (21) de la structure protectrice en forme de disque (20) est constitué d'une matière plastique présentant un module de traction étant supérieur à 0,1 GPa et de préférence supérieur à 1 GPa.

3. Électrode médicale selon la revendication 1 ou 2, dans laquelle le couvercle (21) de la structure protectrice en forme de disque (20) forme une chambre intérieure fermée (24) lorsque la structure protectrice en forme de disque (20) est fixée à l'électrode médicale (1).

4. Électrode médicale selon l'une quelconque des revendications précédentes, dans laquelle le bord extérieur (22) de la structure protectrice en forme de disque (20) est fixé au côté supérieur (8) de la feuille plastique (7).

5. Électrode médicale selon la revendication 4, dans laquelle le bord extérieur (22) de la structure protectrice en forme de disque (20) est fixé au côté supérieur (8) de la feuille plastique (7) au-dessus ou radialement à l'extérieur de la ligne de soudure (10) entourant l'ouverture (5) dans le disque en mousse plastique (2).

6. Électrode médicale selon l'une quelconque des revendications précédentes, dans laquelle une éponge (17) constituée d'un matériau en mousse, tel qu'une mousse à pores ouverts ou une mousse à cellules fermées, est agencée entre le couvercle (21) et le côté supérieur (3) du disque en mousse plastique (2) et/ou la feuille plastique (7).

7. Électrode médicale selon la revendication 6, dans laquelle l'éponge (17) est agencée entre le couvercle (21) et le côté supérieur (3) du disque en mousse plastique (2) dans un état comprimé.

8. Électrode médicale selon la revendication 7, dans laquelle l'éponge (17) dans son état comprimé présente une hauteur comprimée étant inférieure à 50 % d'une hauteur non comprimée de l'éponge (17) avant compression et agencement de l'éponge (17) entre le couvercle (21) et le côté supérieur (3) du disque en mousse plastique (2) et/ou de la feuille plastique (7).

9. Électrode médicale selon l'une quelconque des revendications précédentes, dans laquelle l'électrode médicale (1) inclut un blindage électrostatique (28) couvrant au moins la totalité du côté supérieur (3) du disque en mousse plastique (2), et dans laquelle le blindage électrostatique (28) inclut au moins une couche dissipative supérieure (29) et une couche conductrice inférieure (30).

10. Électrode médicale selon la revendication 9, dans laquelle le blindage électrostatique (28) forme une zone de bord (31) s'étendant depuis un bord (32) du disque en mousse plastique (2), et dans laquelle, dans ladite zone de bord (31), un côté inférieur (33) de la couche conductrice inférieure (30) du blindage électrostatique (28) est pourvu d'une couche de gel solide électroconductrice et adhésive (34).

11. Électrode médicale selon la revendication 9 ou 10, dans laquelle le blindage électrostatique (28) est adapté pour couvrir le connecteur électrique (11).

12. Électrode médicale selon la revendication 11, dans laquelle le blindage électrostatique (28) est pourvu d'au moins un rabat (35) adapté pour être plié autour du connecteur électrique (11) et éventuellement d'une partie d'un câble (12) y étant connecté.

13. Électrode médicale selon l'une quelconque des revendications 9 à 12, dans laquelle la couche dissipative supérieure (29) du blindage électrostatique (28) est du polypropylène orienté (OPP), et dans laquelle la couche conductrice inférieure (30) du blindage électrostatique (28) est formée par de l'encre électroconductrice imprimée sur un côté inférieur de la couche dissipative supérieure (29).
